# EUROPÄISCHE PATENTANMELDUNG

(11) **EP 0 913 150 A1**
(43) Veröffentlichungstag der Anmeldung: **06.05.1999**
(21) Anmeldenummer: 98250289.0
(22) Anmeldetag: 14.08.1998
(51) Int. Cl.: A61K 31/19

(54) **Wirkstoffkombination aus Salicylalkohol und Fumarsäure und/oder Maleinsäure, zur Behandlung von seborrhoischen Hauterkrankungen, insbesondere der Schuppenflechte**

(30) Priorität: 15.08.1997 DE 19736265
(71) Anmelder: Phytexpert, Pharm-Tox-Consulting GmbH, 23627 Gross Sarau (DE)
(72) Erfinder: Siegers, Claus-Peter, 23627 Gross Sarau (DE); Siegers, Claudia, 23564 Lübeck (DE); Pentz, Reinhard, 23564 Lübeck (DE)
(74) Vertreter: UEXKÜLL & STOLBERG

(57) **Zusammenfassung**

Die Erfindung betrifft ein Arzneimittel, insbesondere zur Behandlung von seborrhoischen Hauterkrankungen, das durch einen Gehalt an Salicylalkohol sowie Maleinsäure und/oder Fumarsäure als Wirkstoffkombination gekennzeichnet ist.

## Beschreibung

Die Erfindung betrifft ein Arzneimittel, das sich zur Behandlung von seborrhoischen Hauterkrankungen, insbesondere der Schuppenflechte (Psoriasis) eignet, sowie die Verwendung einer neuen Wirkstoffkombination zur Behandlung dieser Krankheiten.

Auf dem Gebiet der Behandlung seborrhoischer Hauterkrankungen wird ständig nach neuen Arzneimittelwirkstoffen gesucht (siehe z.B. den Übersichtsartikel von van de Kerkhof et al. in H+G, Band 70, 11, 1995, Seiten 791 bis 796 zur Behandlung von Schuppenflechte und dafür geeigneter Wirkstoffe). Ziel der Behandlung ist es, die obere Hornschicht entweder abzulösen (Keratolyse) oder die darunterliegenden Zellen in ihrem Wachstum zu hemmen (Keratoplastik). Alle bisher bekannten Wirkstoffe haben jedoch zahlreiche Nebenwirkungen, die ihre Anwendung beschränken. Dementsprechend besteht weiterhin ein erheblicher Bedarf an neuen Medikamenten zur Behandlung seborrhoischer Hauterkrankungen.

Es wurde nunmehr überraschend gefunden, daß Salicylalkohol und Maleinsäure und/oder Fumarsäure eine Wirkstoffkombination darstellen, die sich sehr gut zur Behandlung von seborrhoischen Hauterkrankungen, insbesondere der Schuppenflechte eignet.

Gegenstand der Erfindung ist dementsprechend ein Arzneimittel, das durch einen Gehalt an Salicylalkohol sowie Maleinsäure und/ oder Fumarsäure als Wirkstoffkombination gekennzeichnet ist.

Gegenstand der Erfindung ist ferner die Verwendung von Salicylalkohol in Kombination mit Maleinsäure und/oder Fumarsäure zur Herstellung eines Arzneimittels zur Bekämpfung von seborrhoischen Hautkrankheiten, insbesondere der Schuppenflechte.

Das Gewichtsverhältnis von Salicylalkohol (2-Hydroxybenzylalkohol) zu Maleinsäure und/oder Fumarsäure kann in Abhängigkeit von der speziellen Applikation in weiten Bereichen schwanken. Üblicherweise liegt es im Bereich von 100 : 1 bis 2 : 1, insbesondere 50 : 1 bis 5 : 1 und vorzugsweise 20 : 1 bis 5 : 1 (z.B. 10 : 1). Maleinsäure und Fumarsäure können als solche oder auch in Form physiologisch verträglicher Salze eingesetzt werden. Bevorzugt sind die freien Säuren, die auch der Berechnung bei den vorgenannten Gewichtsverhältnissen zugrundegelegt sind.

Die Konzentration der erfindungsgemäßen Wirkstoffkombination hängt ebenfalls von der speziellen Applikation und der gewünschten Behandlungsintensität ab. Bei topischen Arzneimittelformen liegt die Konzentration der Wirkstoffkombination üblicherweise im Bereich von 1 bis 20 Gew.-% und vorzugsweise 5 bis 10 Gew.-%.

Je nach vorgesehener Anwendung kann die erfindungsgemäße Wirkstoffkombination mit geeigneten Trägerstoffen, Hilfsmitteln und/oder Zusatzstoffen kombiniert werden. Beispiele für geeignete Träger- und Salbengrundlagen ergeben sich aus den nachfolgenden Beispielen.

Die erfindungsgemäße Wirkstoffkombination eignet sich zur Behandlung von seborrhoischen Hauterkrankungen, insbesondere Schuppenflechte (Psoriasis), Warzen, Schrunden, Milchschorf, Akne vulgaris, chronische Dermatitis und Ekzemen mit Hyperkeratose. Die erfindungsgemäße Wirkstoffkombination zeichnet sich durch eine hohe Verträglichkeit aus. Ein Irritationspotential oder Risiken sind bisher nicht bekannt.

### Beispiel 1

Die erfindungsgemäße Wirkstoffkombination sowie Wirkstoffe, die bereits heute in der klinischen Behandlung der Schuppenflechte eingesetzt werden (siehe Rote Liste 1997), wurden an einem bekannten Zellkulturmodell menschlicher Keratinozyten untersucht. Die Zellproliferation der Hautzellen wurde anhand der bekannten Neutralrotmethode untersucht. Die Zellschädigung wurde durch gleichzeitige Untersuchung der Freisetzung eines cytosolischen Enzyms, der Lactatdehydrogenase, zusätzlich bestimmt. In einigen Versuchen wurde auch die DNA-Synthese anhand der Inkorporation von ³H-Thymidin mituntersucht. Ergebnis dieser Zellkulturuntersuchungen war, daß sich die Kombination aus Salicylalkohol und Maleinsäure bzw. Salicylalkohol und Fumarsäure im Vergleich zu Natriumfumarat, Natriumsalicylat und Dexamethason anhand der sogenannten IC₅₀-Werte, die eine 50%ige Hemmung des Zellwachstums bedeuten, als besonders wirksam erwies. Die erhaltenen Ergebnisse sind in der nachfolgenden Tabelle zusammengefaßt.

**Tabelle:**

| **Hemmung der Zellproliferation (NR), Cytotoxizität (LDH-Freisetzung) und Einfluß auf die DNA-Synthese (**^{**3**}**H-Thymidin-Einbau) an menschlichen Keratinozyten (HaCat)** | | | |
|---|---|---|---|
| **Wirkstoff** | **NR-Methode *IC**_{**50**} **g/l** | **LDH-Freisetzung* g/l** | ^{**3**}**H-Thymidin-Einbau** |
| Na-Fumarat | 4,9 | > 10 | - |
| Na-Salicylat | 1,5 | > 2 | - |
| Maleinsäure | 1,7 | > 1 | - |
| Salicylalkohol | 0,78 | > 0,6 | 0,3 |
| Na-Fumarat + Na-Salicylat | 1,0 | > 2 | - |
| Salicylalkohol + Maleinsäure | 0,3 | > 0,6 | - |
| Dexamethason | 0,8 | > 0,6 | - |
| Salicylalkohol + Na-Fumarat | 0,52 | > 0,6 | - |
| Salicylaldehyd | 0,034 | > 0,1 | - |

| | | | |
|---|---|---|---|
| * als Maß einer Cytotoxizität bezogen auf die Konzentration des Wirkstoffs in g/l | | | |

### Beispiel 2

Eine zur Einreibung von Hautpartien geeignete Salbe, die bei Schuppenflechte oder seborrhoischen Ekzemen eingesetzt werden kann (jedoch nicht im Bereich des Kopfhaares), wurde aus 5 Gew.-% Salicylalkohol, 0,5 Gew.-% Maleinsäure und 94,5 Gew.-% weißer Vaseline in Form einer Verreibung hergestellt.

### Beispiel 3

Es wurde eine zur Behandlung der Schuppenflechte im Kopfhaarbereich geeignete Rezeptur aus 5 Gew.-% Salicylalkohol, 0,5 Gew.-% Maleinsäure, 10 Gew.-% Propylenglykol und 84,5 Gew.-% 40%igem Isopropylalkohol hergestellt.

### Beispiel 4

Es wurde eine alkoholische Tinktur zur Behandlung von Warzen und Schrunden hergestellt, die aus 10 Gew.-% Salicylalkohol, 1 Gew.-% Maleinsäure und 89 Gew.-% 70%igem Ethylalkohol bestand.

### Beispiel 5

Es wurde eine Formulierung aus 5 Gew.-% Salicylalkohol, 0,1 Gew.-% Maleinsäure und 94,9 Gew.-% Eucerinum cum aqua 100 hergestellt. Diese eignet sich zur Behandlung von nicht-behaarten Hautpartien bei trockener, schuppiger Haut, bei Schuppenflechte und seborrhoischen Ekzemen. Die Wirkstoffe sind hier in der hydrophilen Salbengrundlage gelöst, während sie bei der Formulierung gemäß Beispiel 2 nur suspendiert sind.

### Beispiel 6

Die Formulierungen gemäß den Beispielen 2 bis 5 wurden in entsprechender Weise hergestellt, wobei jedoch Maleinsäure ganz oder teilweise gegen Fumarsäure ausgetauscht wurde.

## Patentansprüche

1. Arzneimittel, insbesondere zur Behandlung von seborrhoischen Hauterkrankungen, **gekennzeichnet** durch einen Gehalt an Salicylalkohol sowie Maleinsäure und/oder Fumarsäure oder deren physiologisch verträglichen Salzen als Wirkstoffkombination.

2. Arzneimittel nach Anspruch 1, **dadurch gekennzeichnet**, daß das Gewichtsverhältnis von Salicylalkohol zu Maleinsäure und/oder Fumarsäure im Bereich von 100 : 1 bis 2 : 1, vorzugsweise 20 : 1 bis 5 : 1 liegt.

3. Arzneimittel nach Anspruch 1 oder 2, **dadurch gekennzeichnet**, daß es als topisch anwendbares Arzneimittel, insbesondere in Form einer Salbe vorliegt.

4. Arzneimittel nach Anspruch 3, **dadurch gekennzeichnet**, daß es die Wirkstoffkombination in einer Menge von 1 bis 20 Gew.-%, vorzugsweise 5 bis 10 Gew.-% enthält.

5. Verwendung von Salicylalkohol in Kombination mit Maleinsäure und/oder Fumarsäure oder deren physiologisch verträglichen Salzen zur Herstellung eines Arzneimittels zur Bekämpfung von seborrhoischen Hauterkrankungen, insbesondere der Schuppenflechte.
